# EUROPEAN PATENT APPLICATION

(11) **EP 2 843 576 A1**
(43) Date of publication of application: **04.03.2015**
(21) Application number: 13782493.4
(22) Date of filing: 26.04.2013
(51) Int. Cl.: G06F 19/22, C12Q 1/68

(54) **METHOD FOR DETERMINING READ ERROR IN NUCLEOTIDE SEQUENCE**

(30) Priority: 26.04.2012 JP 2012101755
(71) Applicant: National Institute of Radiological Sciences, Chiba-shi, Chiba 263-8555 (JP); Maze, Inc., Tokyo 193-0835 (JP)
(72) Inventor: ABE, Masumi, Chiba-shi Chiba 263-8555 (JP); KASAMA, Yasuji, Chiba-shi Chiba 263-8555 (JP); YUNOKAWA, Harunobu, Tokyo 193-0835 (JP); SATO, Shinji, Chosei-gun Chiba 299-4336 (JP); KONDO, Kazuhiro, Tokyo 193-0835 (JP); HIEIDA, Takashi, Tokyo 193-0835 (JP)
(74) Representative: Delumeau, François Guy
(86) International application number: PCT/JP2013/062426
(87) International publication number: WO 2013/162010

(57) **Abstract**

A method is a method for determining whether a difference between a first reference sequence and first comparative sequences sharing homology with the first reference sequence is caused by a mutation of the first comparative sequence or caused by a read error in sequencing. The method includes generating second comparative sequences and a second reference sequence by substituting a sequence having a predetermined base number or more of consecutive same bases for a sequence having the predetermined base number of the consecutive same bases in the first comparative sequences and the first reference sequence (S103). The method further includes calculating an edit distance of the second comparative sequence with respect to the second reference sequence (S104), and determining whether the difference is caused by the mutation or caused by the read error based on the edit distance (S105).

## Description

### Technical Field

The present invention relates to a method for determining a read error of a base sequence.

### Background Art

For identifying mutations such as a single nucleotide polymorphism (SNP), substitution of a plurality of bases, insertion or deletion of a base, there is much demand in a study field, a clinical field, or similar field, for example, diagnosis and treatment, identification of virus and bacteria, and analysis on genetic information of livestock or similar information. For example, WO2006/110855 discloses a technique for efficiently identifying a shift of an SNP or similar mutation by sequencing using a pyrophosphate sequencing technique.

### Disclosure of the Invention

For example, base sequence data acquired by sequencing using the pyrophosphate sequencing technique or similar technique as disclose in WO2006/110855 might contain a read error in which the number of the consecutive same bases is different from the actual number in a portion having the consecutive same bases. It is difficult to determine whether the difference between a reference sequence and a comparative sequence to be compared with this reference sequence is caused by a mutation or caused by a read error in sequencing.

Therefore, it is an object of the present invention to provide a method for determining whether the difference in base sequence is caused by a mutation or caused by a read error.

To achieve the above-described object, according to one aspect of the present invention, a method for determining a read error of a base sequence is a method for determining whether a difference between a first reference sequence and a plurality of first comparative sequences sharing homology with the first reference sequence is caused by a mutation of the first comparative sequence or caused by a read error in sequencing. The method includes: generating respective second comparative sequences by substituting a sequence having a predetermined base number or more of consecutive same bases for a sequence having the predetermined base number of the consecutive same bases in the respective first comparative sequences; generating a second reference sequence by substituting a sequence having the predetermined base number or more of consecutive same bases for a sequence having the predetermined base number of the consecutive same bases in the first reference sequence; calculating a plurality of edit distances after shortening as edit distances of the respective second comparative sequences with respect to the second reference sequence; calculating an average value after shortening as an average value of the plurality of edit distances after shortening; and determining whether the difference is caused by the mutation or caused by the read error based on the average value after shortening.

To achieve the above-described object, according to one aspect of the present invention, the method for determining the read error of the base sequence is a method for determining whether a difference between a first reference sequence and a first comparative sequence sharing homology with the first reference sequence is caused by a mutation of the first comparative sequence or caused by a read error in sequencing. The method includes: generating a second comparative sequence by substituting a sequence having a predetermined base number or more of consecutive same bases for a sequence having the predetermined base number of the consecutive same bases in the corresponding first comparative sequence; generating a second reference sequence by substituting a sequence having the predetermined base number or more of consecutive same bases for a sequence having the predetermined base number of the consecutive same bases in the first reference sequence; calculating an edit distance of the second comparative sequence with respect to the second reference sequence; and determining whether the difference is caused by the mutation or caused by the read error based on the edit distance.

The present invention allows providing a method for determining whether a difference in base sequence is caused by a mutation or caused by a read error.

### Brief Description of the Drawings

FIG. 1 is a block diagram illustrating an exemplary configuration of a determination apparatus according to each embodiment of the present invention.
FIG. 2 is a block diagram illustrating an exemplary configuration of a function block of the determination apparatus according to each embodiment of the present invention.
FIG. 3 is a flowchart illustrating an exemplary process by the determination apparatus according to a first embodiment.
FIG. 4 is a flowchart illustrating an exemplary process by the determination apparatus according to a second embodiment.
FIG. 5 is a block diagram illustrating an exemplary configuration of the function block of the determination apparatus according to a modification of the second embodiment.
FIG. 6 is a schematic diagram for describing HiCEP.
FIG. 7 is a flowchart illustrating an exemplary process by a cluster determination apparatus according to a third embodiment.

### Description of Preferred Embodiments

### [First Embodiment]

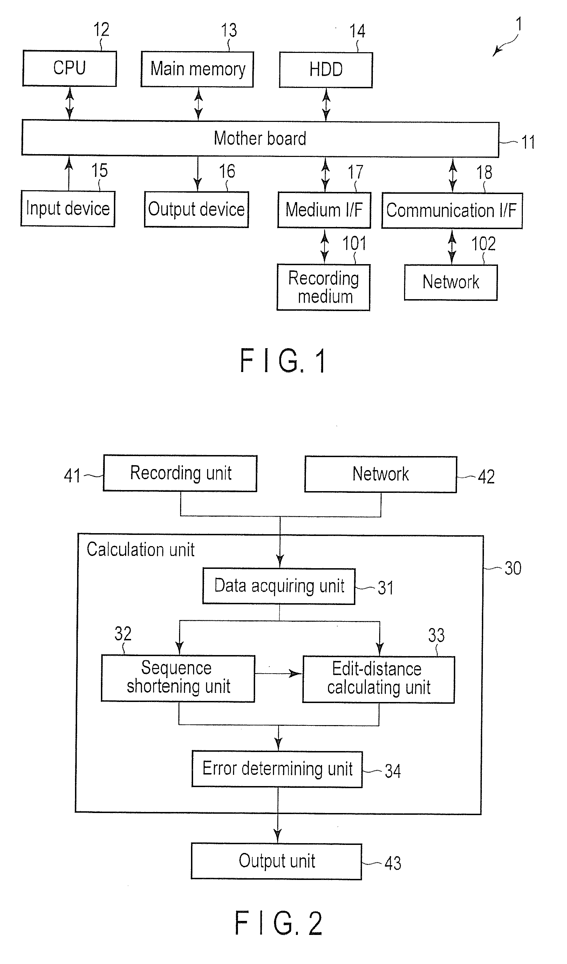

A description will be given of a first embodiment of the present invention with reference to the drawings. FIG. 1 illustrates the outline of an exemplary configuration of a determination apparatus 1 for determining whether the difference in base sequence according to this embodiment is caused by a mutation or a read error. The determination apparatus 1 includes a mother board 11, a CPU 12, a main memory 13, a hard disk drive (HDD) 14, an input device 15, an output device 16, a recording-medium interface (medium I/F) 17, and a communication interface (communication I/F) 18.

Each unit in the determination apparatus 1 communicates with one another through the mother board 11. The CPU 12 executes various operations. The main memory 13 includes, for example, a DRAM and temporarily stores information required for the operation in the CPU 12 or similar information. The HDD 14 stores various information including a program related to the operation executed by the CPU 12 and similar information. The input device 15 includes, for example, user interfaces such as a keyboard and a computer mouse related to input from the user. The output device 16 includes, for example, user interfaces such as a display and a printer related to output to the user. The medium I/F 17 is an interface that communicates with a recording medium 101. The communication I/F 18 is an interface for connecting to a network 102.

FIG. 2 illustrates the outline of an exemplary configuration of the function block of the determination apparatus 1 according to this embodiment. The determination apparatus 1 includes the CPU 12 and a calculation unit 30 configured by a program recorded in the main memory 13 or the HDD 14. The calculation unit 30 includes a data acquiring unit 31, a sequence shortening unit 32, an edit-distance calculating unit 33, and an error determining unit 34. The calculation unit 30 couples to a recording unit 41, a network 42, and an output unit 43.

The data acquiring unit 31 acquires data related to a base sequence from the recording unit 41 that includes the HDD 14, the recording medium 101, and similar member or from the network 42. This data includes a reference sequence and a comparative sequence. The comparative sequence has the difference from the reference sequence. It is necessary to determine whether the difference is caused by a mutation or a read error. The reference sequence acquired by the data acquiring unit 31 is referred to as a first reference sequence. The comparative sequence is referred to as a first comparative sequence.

Regarding the first reference sequence, the sequence shortening unit 32 substitutes the sequence where three or more of the consecutive same bases are arranged for the sequence where two of those consecutive bases are arranged, so as to generate a shortened base sequence. This shortened base sequence is referred to as a second reference sequence. Regarding the first comparative sequence, the sequence shortening unit 32 similarly substitutes the sequence where three or more of the consecutive same bases are arranged for the sequence where two of those consecutive bases are arranged, so as to generate a shortened sequence. This shortened sequence is referred to as a second comparative sequence.

The edit-distance calculating unit 33 calculates the edit distance of each first comparative sequence with respect to the first reference sequence. Similarly, the edit-distance calculating unit 33 calculates the edit distance of each shortened second comparative sequence with respect to the shortened second reference sequence. The error determining unit 34 determines whether the difference between the first reference sequence and the first comparative sequence is caused by a mutation or a read error based on the edit distance calculated by the edit-distance calculating unit 33.

The determination apparatus 1 according to this embodiment performs the following process. For example, in a pyrophosphate sequencing technique using sequencing such as Pyrosequencing (registered trademark) method, a read error in which an error is made in the number of consecutive bases is likely to be contained in the case where there are the consecutive same bases. The problem containing this error is what is called a homopolymer problem. The determination apparatus 1 determines whether the difference between the first reference sequence and the first comparative sequence is caused by the read error related to the homopolymer problem or the presence of a mutation.

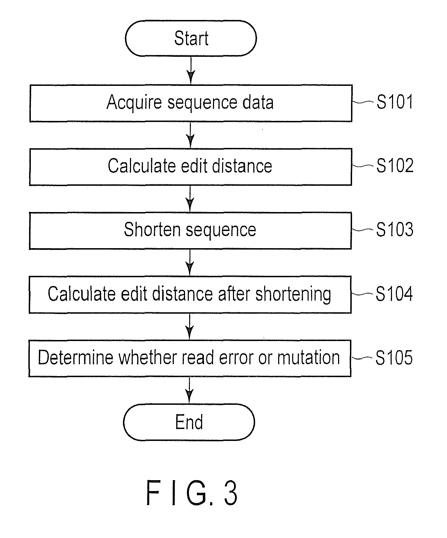

A description will be given of the operation of the determination apparatus 1 according to this embodiment. FIG. 3 illustrates a flowchart of processes executed in this embodiment. In step S101, the data acquiring unit 31 of the calculation unit 30 acquires sequence data. The sequence data includes the first reference sequence and the first comparative sequence. Here, the sequence data includes the first comparative sequence having a difference with respect to the first reference sequence. This difference is identified by a known method. It is unclear whether this difference is a read error in sequencing or a difference due to the presence of a mutation. The first reference sequence and the first comparative sequence are sequences that contain this difference and have a predetermined number of bases. Here, for the first reference sequence, various sequences including the sequence of the transcript, a genome sequence, or similar sequence can be used. The sequence data may be acquired from the recording medium 101 or acquired from the network 102. Alternatively, it is possible to acquire the data of the first reference sequence and the first comparative sequence recorded in the HDD 14.

In this description, for example, the first reference sequence is defined as "AGCCTTTA" (hereinafter referred to as the sequence 1) and the first comparative sequence is defined as "AGCCTTTTA" (hereinafter referred to as the sequence 2). That is, the sequence 1 and the sequence 2 are different in the number of consecutive "T"s.

In step S102, the edit-distance calculating unit 33 of the calculation unit 30 calculates the edit distance of the first comparative sequence with respect to the first reference sequence. Here, the edit distance is the value obtained by comparing the reference sequence with a target sequence to obtain the presence of an insertion, a deletion, or a substitution of the base and by adding "1" for each presence of the insertion, the deletion or the substitution. For example, with respect to the first reference sequence "AGCCTTTA" (sequence 1), the edit distance of the first comparative sequence "AGCCTTTTA" (sequence 2) is 1 because there is one insertion of T.

In step S103, the sequence shortening unit 32 of the calculation unit 30 generates shortened sequences of the first reference sequence and the first comparative sequence by converting the sequence where three or more bases of the consecutive same bases into the sequence where two bases of those consecutive bases. For example, the sequence 1 is converted into "AGCCTTA" (hereinafter referred to as a sequence 3) and the sequence 2 is converted into "AGCCTTA" (hereinafter referred to as a sequence 4). The shortened sequence of the first reference sequence is referred to as the second reference sequence. The shortened sequence of the first comparative sequence is referred to as the second comparative sequence.

In step S104, the edit-distance calculating unit 33 of the calculation unit 30 calculates the edit distance of the second comparative sequence with respect to the second reference sequence. The edit distance of the sequence 4 with respect to the sequence 3 is 0 because both the sequences coincide with each other.

In step S105, the error determining unit 34 of the calculation unit 30 determines whether the difference between the first reference sequence and the first comparative sequence is caused by a mutation or a read error based on the edit distance before the shortening and the edit distance after the shortening. For example, the error determining unit 34 determines that the difference is caused by a shift when the edit distance before the shortening and the edit distance after the shortening are the same, and determines the difference is caused by a read error when the edit distance before the shortening and the edit distance after the shortening are different. It may be determined that the difference is caused by a read error when the edit distance after the shortening is 0, and it may be determined that the difference is caused by a mutation when the edit distance after the shortening is other than 0. In the example of the sequence 1 and the sequence 2, the edit distance before the shortening is 1 and the edit distance after the shortening is 0. Therefore, it is determined that the difference between the sequence 1 and the sequence 2 is caused by a read error. The calculation unit 30 outputs the determination result to the output unit 43 including the HDD 14, the output device 16, the recording medium 101, the network 102, and similar member so as to, for example, record the determination result. Then, the process terminates.

The read error related to the homopolymer problem hardly occurs even when two of the consecutive same bases appear. When three or more of the consecutive same bases appear, the occurrence rate of the error increases as the number of consecutive bases is larger. Therefore, in this embodiment, when the consecutive same bases appear, all the numbers of consecutive bases are shortened to two such that the analysis is not affected by a read error in sequencing. That is, in the case where there is a read error in sequencing caused by the homopolymer problem, the difference between the sequences is eliminated by shortening. In the case where there is a mutation without a read error, the difference is maintained by shortening. These conditions are used.

Another example of the first comparative sequence will be described. The first reference sequence is defined as the sequence 1, and the first comparative sequence is defined as "AGCCGTTA" (hereinafter referred to as a sequence 5). At this time, the edit distance of the sequence 5 with respect to the sequence 1 calculated in step S102 is 1 because one of "T" is substituted for "G." The second comparative sequence obtained by shortening the sequence 5 in step S103 is "AGCCGTTA" (hereinafter referred to as a sequence 6). The edit distance of the sequence 6 with respect to the sequence 1 calculated in step S104 is 1. Accordingly, in step S105, it is determined that there is a mutation.

As described above, according to this embodiment, a homopolymer is shortened in the analysis of the sequence data containing the homopolymer problem. Based on the edit distance of the shortened sequence, it is determined that the difference in the sequence is caused by a mutation or a read error. That is, this embodiment allows accurately identifying an SNP or similar mutation.

The processes described with reference to FIG. 3 are one example. The order in the respective processes can be changed, or a part of the processes can be changed or omitted. While in this embodiment the sequence having three or more of the consecutive same bases is shortened to two of the bases, the sequence may be shortened to any number. For example, the sequence having four or more of the consecutive same bases may be shortened to three bases. However, the occurrence rate of the read error increases in the case where there are three or more of the consecutive bases. Therefore, it is preferred to shorten the sequence having three or more of the consecutive same bases to two of the bases.

### [Second Embodiment]

A second embodiment of the present invention will be described. Here, the difference from the first embodiment will be described. Like reference numerals designate corresponding or identical elements, and therefore such elements will not be further elaborated here. In the sequencing using the pyrophosphate sequencing technique, a large amount of sequence data is obtained at once. In this embodiment, a group of sequences sharing homology with a reference sequence is treated as sequence data. The determination apparatus 1 according to this embodiment determines whether the difference is caused by a read error or by a heterozygous SNP or similar mutation in a portion having the difference from the reference sequence in this group of sequences. Here, the SNP or similar mutation denotes a substitution, an insertion or a deletion of one base. Like the homopolymer problem, in the case where the read error occurs not randomly but under a certain condition, the read error might be determined as the SNP or similar mutation by a conventional determination method. In this embodiment, this read error containing the homopolymer problem is discriminated. Here, in the case where there is a heterozygous SNP or similar mutation, the average value of the edit distances of the respective sequences with respect to the reference sequence ideally becomes 0.5. Accordingly, there are two types of sequences at the respective rates of 50%.

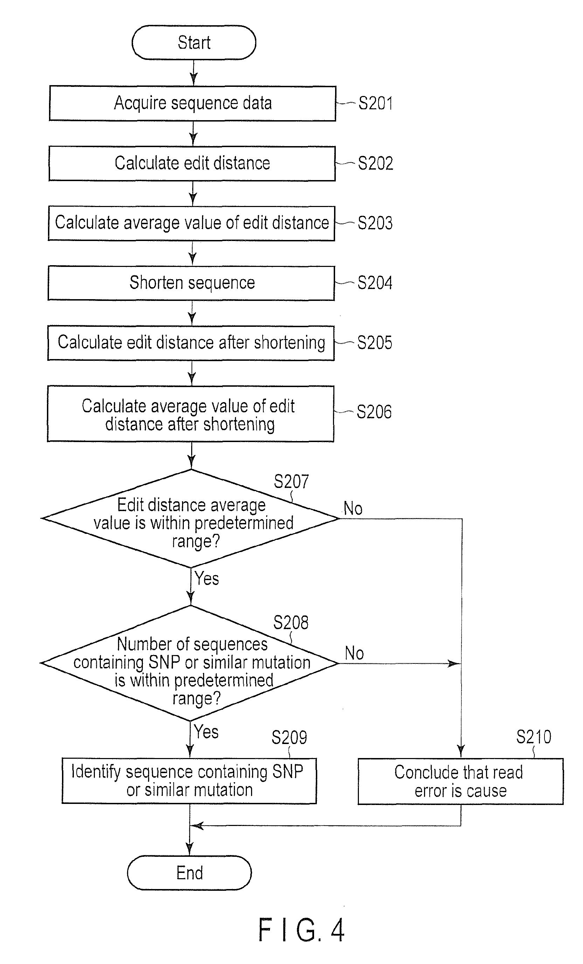

A description will be given of the operation of the determination apparatus 1 according to this embodiment. FIG. 4 illustrates a flowchart of processes executed in this embodiment. In step S201, the data acquiring unit 31 of the calculation unit 30 acquires sequence data. The sequence data is the sequence in a portion having the difference from the reference sequence in a group of sequences considered to share homology with the reference sequence. For the reference sequence, various sequences including the sequence of the transcript, a genome sequence, or similar sequence can be used. Table 1 shows one example of the sequence data. The topmost row of Table 1 shows a reference sequence as the first reference sequence. Beneath the topmost row, sequences as the first comparative sequences are shown. These sequences are referred to as a sequence 7 to a sequence 14. In the sequences 7 to 14, the portion different from the reference sequence is the difference that is not determined to be caused by a read error in sequencing or by an SNP or mutation.

**Table 1**

| Reference sequence | AGCCT-TTA | Edit distance |
|---|---|---|
| Sequence 7 | AGCCT-TTA | 0 |
| Sequence 8 | AGCCT-TTA | 0 |
| Sequence 9 | AGCCT-TTA | 0 |
| Sequence 10 | AGCCTTTTA | 1 |
| Sequence 11 | AGCCTTTTA | 1 |
| Sequence 12 | AGCC--TTA | 1 |
| Sequence 13 | AGCC--TTA | 1 |
| Sequence 14 | AGCCG-TTA | 1 |

In step S202, the edit-distance calculating unit 33 of the calculation unit 30 calculates the edit distances of the respective first comparative sequences with respect to the first reference sequence. For example, the edit distance of the respective sequences shown in Table 1 is calculated as follows. Since the respective sequences 7 to 9 are the same as a consensus sequence, the respective edit distances are 0. In the sequences 10 and 11, since one of T is inserted into the consensus sequence, the edit distance is 1. In the sequence 12 and 13, since one of T is deleted from the consensus sequence, the edit distance is 1. In the sequence 14, since T is substituted for G in the consensus sequence, the edit distance is 1. In step S203, the edit-distance calculating unit 33 of the calculation unit 30 calculates the average value of the edit distances of all the first comparative sequences.

In step S204, the sequence shortening unit 32 of the calculation unit 30 generates shortened sequences of the first reference sequence and the respective first comparative sequences by converting the sequence where three or more bases of the consecutive same bases into the sequence where two of those consecutive bases. For example, the sequences 7 to 14 as the first comparative sequences shown in Table 1 are converted into respective sequences 15 to 22 as the shortened second comparative sequences shown in Table 2.

**Table 2**

| Reference sequence | AGCC-TTA | Edit distance |
|---|---|---|
| Sequence 15 | AGCC-TTA | 0 |
| Sequence 16 | AGCC-TTA | 0 |
| Sequence 17 | AGCC-TTA | 0 |
| Sequence 18 | AGCC-TTA | 0 |
| Sequence 19 | AGCC-TTA | 0 |
| Sequence 20 | AGCC-TTA | 0 |
| Sequence 21 | AGCC-TTA | 0 |
| Sequence 22 | AGCCGTTA | 1 |

In step S205, the edit-distance calculating unit 33 of the calculation unit 30 calculates the edit distances of the respective second comparative sequences with respect to the second reference sequence. This edit distances are, for example, as shows in Table 2. In step S206, the edit-distance calculating unit 33 of the calculation unit 30 calculates the average value of the edit distances of the second comparative sequences.

In step S207, the error determining unit 34 of the calculation unit 30 determines whether the difference between the first reference sequence and the first comparative sequence might be caused by an SNP or similar mutation or by the read error based on whether or not the average value of the edit distances of the second comparative sequences is within a predetermined range. For example, the determination is made based on determination criteria shown in Table 3. That is, in the case where the average value of the edit distances of the second comparative sequences (after the shortening) is equal to or more than 0.25 and equal to or less than 0.75, it is determined that an SNP or similar mutation might be the cause. This is based on the fact that the edit distance is ideally 0.5 in the case of a heterozygous diploid in which there is an SNP or similar mutation in one allele. Here, while in this embodiment the range of the average value of the edit distances is set to be equal to or more than 0.25 and equal to or less than 0.75 as the value having 0.5 in the center, another value including 0.5 may be set.

For the reason described later in detail, in the case where the average value of the edit distances of the first comparative sequences (before the shortening) is equal to or more than 0.25 and equal to or less than 0.75 even when the average value of the edit distances of the second comparative sequences (after the shortening) is larger than 0.75, it is determined that an SNP or similar mutation might be the cause. Otherwise, it is determined that a read error is the cause. When it is determined that an SNP or similar mutation might be the cause in step S207, the process proceeds to step S208. When it is determined that a read error is the cause, the process proceeds to step S210.

**Table 3**

| | | Before shortening | | |
|---|---|---|---|---|
| | | Edit distance < 0.25 | 0.25 ≤ Edit distance ≤ 0.75 | 0.75 < Edit distance |
| After shortening | Edit distance < 0.25 | Read error | Read error | Read error |
| | 0.25 ≤ Edit distance ≤ 0.75 | SNP | SNP | SNP |
| | 0.75 < Edit distance | Read error | SNP | Read error |

In step S208, the error determining unit 34 of the calculation unit 30 determines whether or not the proportion of the number of sequences that might have an SNP or similar mutation is within a predetermined range so as to determine whether the difference between the first reference sequence and the first comparative sequence might be caused by an SNP or similar mutation or is caused by a read error. For example, when the number of the sequences that might have an SNP or similar mutation is 33% or more and 67% or less of the number of all sequences, it is determined that the difference is caused by an SNP or similar mutation. Otherwise, it is determined that the difference is caused by a read error. This determination may be changed as follows. When the second comparative sequences are categorized into respective groups of the same sequences, in the case where the number of sequences in the group including the largest number of sequences is 66% or less of the number of all the sequences and the number of sequences in the group including the second largest number of sequences is 33% or more of the number of all the sequences, it is determined that the difference is caused by an SNP or similar mutation.

When it is determined that the difference is caused by an SNP or similar mutation, the process proceeds to step S209. When it is determined that the difference is caused by a read error, the process proceeds to step S210. That is, when the condition of the determination in step S207 and the condition of the determination in step S208 are both satisfied, the process proceeds to step S209. Otherwise, the process proceeds to step S210.

In step S209, the error determining unit 34 of the calculation unit 30 identifies the sequence that having an SNP or similar mutation. The calculation unit 30 outputs information related to the sequence having the SNP or similar mutation to the output unit 43 so as to, for example, record the information. Then, the process terminates.

On the other hand, in step S210, the error determining unit 34 of the calculation unit 30 concludes that the difference between the first reference sequence and the first comparative sequence is caused by a read error and outputs the result to the output unit 43 so as to, for example, record the result. Then, the process terminates.

In the first example of the clusters shown in Table 1, in step S205, the edit distances of the respective second comparative sequences after the shortening are as shown in Table 2. In this case, the average value of the edit distances of the second comparative sequences after the shortening to be calculated in step S206 is 0.125. Accordingly, in the determination in step S207, it is determined that there is a read error. In step S210, a read error is concluded to be present. Then, the process terminates.

In the case of the example shown in Table 1, with this embodiment, the sequence shortening unit 32 shortens the sequence so as to exclude the error caused by the homopolymer problem, and thus concludes that the difference between the first reference sequence and the first comparative sequence is caused by a read error.

Table 4 shows another example of a group of the first comparative sequences. In this case, the edit distances calculated in step S202 are as shown in Table 4. The average value of the edit distances calculated in step S203 is 0.625.

**Table 4**

| Reference sequence | AGCC-TTTA | Edit distance |
|---|---|---|
| Sequence 23 | AGCC-TTTA | 0 |
| Sequence 24 | AGCC-TTTA | 0 |
| Sequence 25 | AGCCTTTTA | 1 |
| Sequence 26 | AGCCTTTTA | 1 |
| Sequence 27 | AGCC--TTA | 1 |
| Sequence 28 | AGCCGTTTA | 1 |
| Sequence 29 | AGCC-GTTA | 1 |
| Sequence 30 | AGCC-GTTA | 1 |

As a result of shortening in step S204, sequences 23 to 30 are changed into respective sequences 31 to 38 as the second comparative sequences shown in Table 5. In this case, the edit distances after the shortening to be calculated in step S205 are as shown in Table 5. The average value of the edit distances after the shortening to be calculated in step S206 is 0.375.

**Table 5**

| Reference sequence | AGCC-TTA | Edit distance |
|---|---|---|
| Sequence 31 | AGCC-TTA | 0 |
| Sequence 32 | AGCC-TTA | 0 |
| Sequence 33 | AGCC-TTA | 0 |
| Sequence 34 | AGCC-TTA | 0 |
| Sequence 35 | AGCC-TTA | 0 |
| Sequence 36 | AGCCGTTA | 1 |
| Sequence 37 | AGCCGTTA | 1 |
| Sequence 38 | AGCCGTTA | 1 |

In this case, in the determination in step S207, since the average value of the edit distances after the shortening is equal to or more than 0.25 and equal to or less than 0.75, it is determined that an SNP or similar mutation might be present. Then, the process proceeds to step S208. According to the sequences after the shortening shown in Table 5, sequences 36 to 38 are sequences that might have an SNP or similar mutation. Here, 3 as the number of the sequences is 37.5% of 8 as the number of all the sequences. Accordingly, it is also determined that an SNP or similar mutation is present in step S208. The process proceeds to step S209.

Step S209 identifies the fact that the sequences 28 to 30 corresponding to the sequences 36 to 38 have an SNP or similar mutation in the first comparative sequences shown in Table 4. Like this example, this embodiment allows accurately identifying the sequence having an SNP or similar mutation also in the first comparative sequences having the homopolymer problem.

Table 6 shows another example of the group of the first comparative sequences. In this case, the edit distances of sequences 39 to 46 to be calculated in step S202 are as shown in Table 6. The average value of the edit distances to be calculated in step S203 is 0.625.

**Table 6**

| Reference sequence | AGCCTCTTA | Edit distance |
|---|---|---|
| Sequence 39 | AGCCTCTTA | 0 |
| Sequence 40 | AGCCTCTTA | 0 |
| Sequence 41 | AGCCTCTTA | 0 |
| Sequence 42 | AGCCTCTTA | 0 |
| Sequence 43 | AGCCTTTTA | 1 |
| Sequence 44 | AGCCTTTTA | 1 |
| Sequence 45 | AGCCT-TTA | 1 |
| Sequence 46 | AGCC--TTA | 2 |

As a result of shortening in step S204, the sequences 39 to 46 are changed into respective sequences 47 to 54 as the second comparative sequences shown in Table 7. In this case, the edit distances after the shortening to be calculated in step S205 are as shown in Table 7. The average value of the edit distances after the shortening to be calculated in step S206 is 1.

**Table 7**

| Reference sequence | AGCCTCTTA | Edit distance |
|---|---|---|
| Sequence 47 | AGCCTCTTA | 0 |
| Sequence 48 | AGCCTCTTA | 0 |
| Sequence 49 | AGCCTCTTA | 0 |
| Sequence 50 | AGCCTCTTA | 0 |
| Sequence 51 | AGCC--TTA | 2 |
| Sequence 52 | AGCC--TTA | 2 |
| Sequence 53 | AGCC--TTA | 2 |
| Sequence 54 | AGCC--TTA | 2 |

In this example, in the determination in step S207, the average value of the edit distances after the shortening is equal to or more than 0.75 and the average value of the edit distances before the shortening is equal to or more than 0.25 and equal to or less than 0.75. Accordingly, it is determined that the SNP or similar mutation might be contained. The process proceeds to step S208. According to the second comparative sequences after the shortening shown in Table 7, the sequences 51 to 54 are sequences that might have an SNP or similar mutation. Here, 4 as the number of the sequences is 50% of 8 as the number of all the sequences. Accordingly, it is determined that an SNP or similar mutation is present. The process proceeds to step S209. Step S209 identifies the fact that the sequences 43 to 46 corresponding to the sequences 51 to 54 have an SNP or similar mutation in the first comparative sequences shown in Table 6.

Like this example, in the case where there is a substitution of one base in between the sequence having the consecutive same bases, the edit distance after the shortening becomes considerably large. Accordingly, making the determination whether or not separation is necessary based only on the determination whether or not the edit distance after the shortening is equal to or more than 0.25 and equal to or less than 0.75 causes an incorrect result. Thus, in this embodiment, also in the case where the edit distance after the shortening is equal to or more than 0.75 and the edit distance before the shortening is equal to or more than 0.25 and equal to or less than 0.75, it is determined that separation is necessary. This accurately identifies an SNP or similar mutation.

Table 8 shows another example of the group of the first comparative sequences. In this case, the edit distances of the sequences 55 to 62 to be calculated in step S202 is as shown in Table 8. The average value of the edit distances to be calculated in step S203 is 0.125.

**Table 8**

| Reference sequence | AGCTTCTTA | Edit distance |
|---|---|---|
| Sequence 55 | AGCTTCTTA | 0 |
| Sequence 56 | AGCTTCTTA | 0 |
| Sequence 57 | AGCTTCTTA | 0 |
| Sequence 58 | AGCTTCTTA | 0 |
| Sequence 59 | AGCTTCTTA | 0 |
| Sequence 60 | AGCTTCTTA | 0 |
| Sequence 61 | AGCTTCTTA | 0 |
| Sequence 62 | AGCTTTTTA | 1 |

The second comparative sequences as the result by shortening the sequences 55 to 62 in step S204 are changed into respective sequences 63 to 70 shown in Table 9. In this case, the edit distances after the shortening to be calculated in step S205 are as shown in Table 9. The average value of the edit distances after the shortening to be calculated in step S206 is 0.375.

**Table 9**

| Reference sequence | AGCTTCTTA | Edit distance |
|---|---|---|
| Sequence 63 | AGCTTCTTA | 0 |
| Sequence 64 | AGCTTCTTA | 0 |
| Sequence 65 | AGCTTCTTA | 0 |
| Sequence 66 | AGCTTCTTA | 0 |
| Sequence 67 | AGCTTCTTA | 0 |
| Sequence 68 | AGCTTCTTA | 0 |
| Sequence 69 | AGCTTCTTA | 0 |
| Sequence 70 | AGC---TTA | 3 |

In this case, in the determination in step S207, the average value of the edit distances after the shortening is equal to or more than 0.25 and equal to or less than 0.75. Accordingly, it is determined that an SNP or similar mutation might be present. Then, the process proceeds to step S208. According to the sequences after the shortening shown in Table 9, for the first comparative sequence, the sequence 70 is the sequence that might have an SNP or similar mutation. Here, 4 as the number of the sequences is 12.5% of 8 as the number of all the sequences. Accordingly, it is determined that a read error is present. Then, the process proceeds to step S210. Step S210 concludes that a read error is present. Then, the process terminates.

Like this example, even when the average value of the edit distances after the shortening is equal to or more than 0.25 and equal to or less than 0.75 and it is determined that an SNP might be present in step S207, in the case where the number of sequences is considerably biased, it is determined that a read error is present in the determination in step S208. Accordingly, it can be concluded that the difference is caused by a read error.

Thus, according to this embodiment, in the analysis of the sequence data containing the homopolymer problem, the homopolymer is shortened. Based on the edit distance of the shortened sequence, it is accurately determined whether the difference between the first reference sequence and the first comparative sequence is caused by an SNP or similar mutation or is caused by a read error. That is, this embodiment allows accurately identifying an SNP or similar mutation.

Here, the processes described with reference to FIG. 3 are one example. The order in the respective processes can be changed, or a part of the processes can be changed or omitted. This embodiment assumes the case of a heterozygous diploid and the case where approximately half of the first comparative sequences is the same as the reference sequence and an SNP or similar mutation is present in the remaining sequences. Accordingly, in the determination in step S207, the range as the criteria of the determination is set to the range having 0.5 in the center as shown in Table 3. However, this should not be construed in a limiting sense. The case of a homozygous diploid assumes that almost all the first comparative sequences are the sequences having one different base compared with the reference sequence. Accordingly, in the case where there is an SNP or similar mutation, the average value of the edit distances is assumed to be close to 1. Accordingly, in this case, the determination criteria in step S207 corresponding to Table 3 is, for example, as shown in Table 10. In this case, the determination criteria in step S208 is set so as to, for example, determine that the first comparative sequence contains an SNP or similar mutation when 75% or more of the sequences is considered to contain an SNP or similar mutation and determine that the difference of the sequence is caused by a read error when that proportion is less than 75%.

**Table 10**

| | | Before shortening | | |
|---|---|---|---|---|
| | | Edit distance < 0.75 | 0.75 ≤ Edit distance ≤ 1.25 | 1.25 < Edit distance |
| After shortening | Edit distance < 0.75 | Read error | Read error | Read error |
| | 0.75 ≤ Edit distance ≤ 1.25 | SNP | SNP | SNP |
| | 1.25 < Edit distance | Read error | SNP | Read error |

Here, also in case of a heterozygous diploid and in the case where approximately half of the first comparative sequences is different from the reference sequence and contains a first SNP while the remaining sequences contain another second SNP, the determination criteria in step S207 corresponding to Table 3 is, for example, as shown in Table 10. In the determination in step S208, setting is done so as to determine that an SNP or similar mutation is contained in the case where the number of the first comparative sequences containing the first SNP and the number of the second comparative sequences containing the second SNP are each 33% or more and 67% or less of the number of all the sequences and otherwise determine that the difference of the sequence is caused by a read error.

For use other than the identification of an SNP or similar mutation, the configuration with three or more categories of the group by the mutation is possible. In this case, the determination criteria in step S207 and the determination criteria in step S208 become different. For example, in the case of the categorization into four groups, the range of the determination criteria in step S207 is set to the range having 0.25 in the center.

While in this embodiment the edit distance with respect to the reference sequence is obtained, the edit distance with respect to the consensus sequence of a group of sequences may be obtained. Here, the consensus sequence means the sequence determined so as to have the highest identity with respect to all of the group of the first comparative sequences, that is, the sequence in common between a large number of the first comparative sequences.

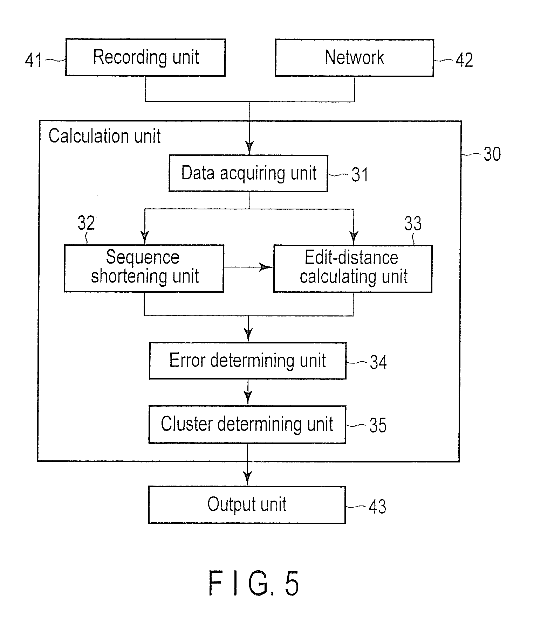

The calculation unit 30 of the determination apparatus 1 may further include a cluster determining unit 35 as illustrated in FIG. 5. This cluster determining unit 35 separates the first comparative sequence into two clusters based on the SNP or similar mutation identified by the error determining unit 34. For example, the cluster determining unit 35 categorizes the group of sequences shown in Table 4 into groups of a first cluster including the sequences 23 to 27 and a second cluster including the sequences 28 to 30. This process is, for example, performed after step S209.

Thus, according to this embodiment, it is determined whether the difference in sequence is caused by a mutation or a read error in sequencing. Using this determination allows determining if variation of genomes or cDNA sequences is true. Additionally, this allows determining if the mutated portion of the cluster generated by assembly of the sequence is true. Additionally, this allows dividing the cluster depending on the determination result, and this can be a pretreatment method for generating clusters.

### [Third Embodiment]

A third embodiment of the present invention will be described. Here, the difference from the second embodiment will be described. Like reference numerals designate corresponding or identical elements, and therefore such elements will not be further elaborated here. In this embodiment, the method for identifying the SNP or similar mutation according to the second embodiment and a method for generating clusters using this method are applied to a high coverage expression profiling (HiCEP) method. Accordingly, the determination apparatus 1 according to this embodiment has the configuration shown in FIG. 5.

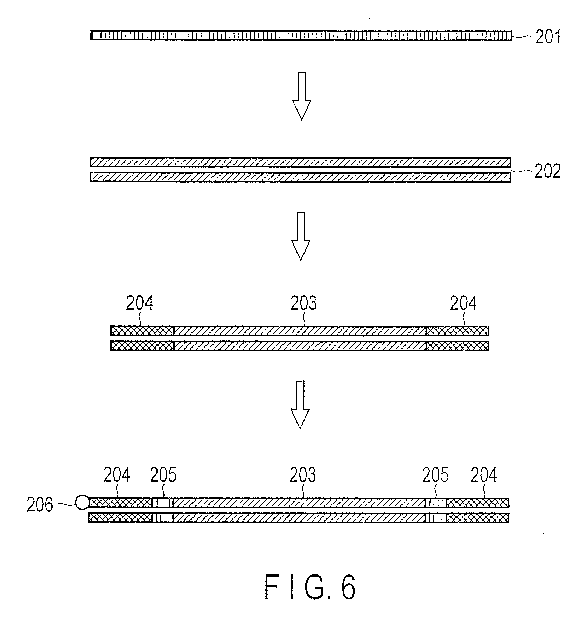

The outline of the HiCEP will be described with reference to FIG. 6. In the HiCEP, an mRNA 201 within a sample is reverse transcripted so as to synthesize various cDNAs 202. As a part of this cDNA, a fragment DNA 203 is cut out. To each end of the fragment DNA 203, an adaptor sequence 204 as an already-known sequence is added. The sample including various fragment DNAs 203 with the addition of these adaptor sequences 204 are divided into 256 pieces. Afterwards, using a PCR method with the adaptor sequence causes amplification of the DNA where two selection bases 205 are inserted into between the fragment DNA 203 and the adaptor sequence 204. Then, a fluorescent dye 206 is added. The dispensation sample divided into 256 pieces has different selection sequences for each dispensation sample. Here, as the selection sequences, two bases are added to each end, that is, four bases in total are added. The number of combinations of the selection sequences is 256. In the HiCEP, 256 types of dispensation samples are each electrophoresed. The fragment DNA 203 included in the dispensation sample is separated per base length. The amount of the fragment DNA 203 is determined using the fluorescent dye 206.

With the HiCEP as described above, regarding 30 to 60 thousand types of fragments of the mRNA in the sample, each of information related to the number of molecules can be acquired. The HiCEP allows exhaustively acquiring a highly-reproducible expression profile with high sensitivity without requiring a genetic resource.

For example, when the fragment whose expression varies is detected by the HiCEP, it is required to identify the gene of this fragment. For exhaustive DNA sequencing, a next-generation sequencer at a high throughput is used. When the homopolymer problem that occurs in sequencing particularly using the pyrophosphate sequencing technique in the next-generation sequencer occurs in the selection base portion adjacent to the adaptor sequence, this problem might negatively affect the analysis using the HiCEP. Thus, in this embodiment, the method for identifying the SNP or similar mutation according to the second embodiment is used for the selection base portion.

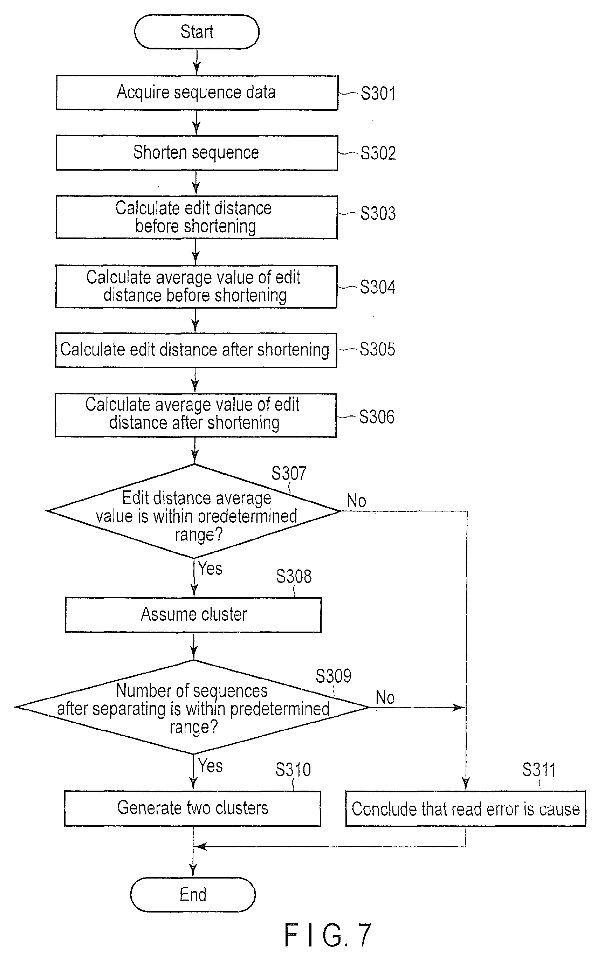

FIG. 7 illustrates a flowchart of a process executed in this embodiment. The data acquiring unit 31 of the calculation unit 30 acquires sequence data in step S301. The sequence data acquired here is, for example, data as shown in Table 11. In this example, a sequence 71 to a sequence 78 are included as the first comparative sequences. As the reference sequence, the consensus sequence of the sequences 71 to 78 is used. This consensus sequence is referred to as a first consensus sequence. In Table 12, the two bases ("TT") on the most read sequence side of the adaptor sequence are described in the row of "Adaptor Sequence" in the table. The sequence on the read sequence side with respect to the adaptor sequence is described in the row of "Read Sequence and Selection Base" in the table.

**Table 11**

| | Read sequence and selection base | Adaptor sequence | Edit distance |
|---|---|---|---|
| Consensus sequence | CAGCCT- | TT | |
| Sequence 71 | CAGCCT- | TT | 0 |
| Sequence 72 | CAGCCT- | TT | 0 |
| Sequence 73 | CAGCCTT | TT | 1 |
| Sequence 74 | CAGCCTT | TT | 1 |
| Sequence 75 | CAGCCCT | TT | 1 |
| Sequence 76 | CAGCCG- | TT | 1 |
| Sequence 77 | CAGCCGT | TT | 1 |
| Sequence 78 | CAGCCGT | TT | 1 |

In step S302, regarding the first consensus sequence and the respective first comparative sequences, the sequence shortening unit 32 of the calculation unit 30 shortens the sequence having three or more bases of the consecutive same bases to a sequence having two bases of the consecutive same bases. The sequences after shortening the sequences shown in Table 11 are shown in Table 12. The sequences 71 to 78 are shortened to respective sequences 79 to 86.

**Table 12**

| | Read sequence and selection base | Adaptor sequence | Edit distance |
|---|---|---|---|
| Consensus sequence | CAGCC | TT | |
| Sequence 79 | CAGCC | TT | 0 |
| Sequence 80 | CAGCC | TT | 0 |
| Sequence 81 | CAGCC | TT | 0 |
| Sequence 82 | CAGCC | TT | 0 |
| Sequence 83 | CAGCC | TT | 0 |
| Sequence 84 | CAGCCG | TT | 1 |
| Sequence 85 | CAGCCG | TT | 1 |
| Sequence 86 | CAGCCG | TT | 1 |

In step S303, the edit-distance calculating unit 33 of the calculation unit 30 calculates the edit distance of the first comparative sequence before the shortening with respect to the first consensus sequence. In step S304, the edit-distance calculating unit 33 of the calculation unit 30 calculates the average value of the edit distances of the first comparative sequences before shortening. In step S305, the edit-distance calculating unit 33 of the calculation unit 30 calculates the edit distance of the second comparative sequence after the shortening with respect to the second consensus sequence generated by shortening the first consensus sequence. In step S306, the edit-distance calculating unit 33 of the calculation unit 30 calculates the average value of the edit distances of the second comparative sequences after the shortening.

Here, for the edit distance, five bases closest to the adaptor sequence in the read sequence and the selection base in the second consensus sequence are set as targets. The edit distances of the second comparative sequences corresponding to these five bases are calculated. That is, the edit distance is calculated regarding the base sequences shown in "Read Sequence and Selection Base" in Table 11 and Table 12. Since the attention is focused on the sequence corresponding to the consensus sequence, the target sequences are not limited to five bases, for example, like the sequences 84 to 86. The edit distances are as shown in Table 11 and Table 12.

In step S307, the error determining unit 34 of the calculation unit 30 determines whether or not the first comparative sequence might contain an SNP or similar mutation corresponding to whether or not the average value of the edit distances is within a predetermined range. For this determination, for example, the determination criteria shown in above-described Table 3 is used. That is, in the case where the edit distance after the shortening is equal to or more than 0.25 and equal to or less than 0.75, it is determined that the first comparative sequence might contain an SNP or similar mutation. Even when the edit distance after the shortening is larger than 0.75, in the case where the edit distance before the shortening is equal to or more than 0.25 and equal to or less than 0.75, it is determined that the first comparative sequence might contain an SNP or similar mutation. Otherwise, it is determined that it is determined that the difference is caused by a read error. When it is determined that the first comparative sequence might contain an SNP or similar mutation, the process proceeds to step S308. When it is determined that the difference is caused by a read error, the process proceed to step S311.

In step S308, when the cluster determining unit 35 of calculation unit 30 assumes the manner of separation when the first base sequence is separated based on two bases on the most adaptor sequence side of the read sequence. This embodiment is focused only on the two bases on the most adaptor sequence side of the read sequence. This is because there is a selection base in this portion and the emphasis is on this selection base. For example, in the example in Table 12, there are two types of "CC" in the sequence 79 to 83 and "CG" in the sequences 84 to 86. Accordingly, the cluster determining unit 35 assumes that the sequences 79 to 86 are separated into two of the cluster including the sequences 79 to 83 and the cluster including the sequences 84 to 86.

In step S309, the error determining unit 34 of the calculation unit 30 determines whether or not the first comparative sequence might contain an SNP or similar mutation corresponding to whether or not the number of sequences included in each cluster is within a predetermined range in the case where the first comparative sequence is separated into two portions. For example, when two clusters are obtained by separation, in the case where the respective numbers of sequences in both the clusters are equal to or more than 33% and equal to or less than 67% of the number of all the sequences, it is determined that the first comparative sequence contains an SNP or similar mutation. This determination may be changed as follows. When the first comparative sequences are categorized into respective groups of the same sequences, in the case where the number of sequences in the group including the largest number of sequences is 66% or less of the number of all the sequences and the number of sequences in the group including the second largest number of sequences is 33% or more of the number of all the sequences, it is determined that an SNP or similar mutation is contained. Otherwise, it is determined that the difference in sequence is caused by a read error. When it is determined that the first comparative sequence contains an SNP or similar mutation, the process proceeds the w step S310. When it is determined that the difference in sequence is caused by a read error, the process proceeds to step S311.

In the example shown in Table 12, the number of sequences in the cluster including the sequences 79 to 83 is 5 that is 62.5% of 8 as the number of all the sequences. On the other hand, the number of sequences in the cluster including the sequences 84 to 86 is 3 that is 37.5% of 8 as the number of all the sequences. Accordingly, it is determined that the first comparative sequence contains an SNP or similar mutation. Then, the process proceeds to step S310.

In step S311, the cluster generating unit 35 of the calculation unit 30 separates the first comparative sequences into the clusters assumed in step S308 so as to generate two clusters. The calculation unit 30 outputs information related to the generated clusters to the output unit 43 so as to, for example, record the information. Then, the process terminates.

In step S311, the cluster determining unit 35 of the calculation unit 30 concludes that difference between the first consensus and the first comparative sequence is caused by a read error. The calculation unit 30 outputs a notification indicating that the difference between the first consensus and the first comparative sequence is caused by a read error to the output unit 43 so as to, for example, record the notification. Then, the process terminates.

This embodiment allows solving the homopolymer problem that become a problem also in analysis using the HiCEP, thus accurately performing clustering in the selection base portion that is important in the HiCEP. Here, this embodiment is not limited to the HiCEP and can be similarly used in another analysis using the adaptor sequence.

### [Fourth Embodiment]

A fourth embodiment of the present invention will be described. Here, the difference from the first embodiment will be described. Like reference numerals designate corresponding or identical elements, and therefore such elements will not be further elaborated here. In this embodiment, similarly to the process in step S103 in the first embodiment, a second base sequence is generated. In the second base sequence, the sequence having three or more of the consecutive same bases is shortened to be converted into the sequence having two of those bases. Based on this second base sequence, an already-known clustering process is performed.

This embodiment shortens the sequence having the consecutive same bases to have a short base length. This improves the efficiency of clustering. Additionally, the sequence having the consecutive same bases is shortened so as to solve the homopolymer problem. This improves the accuracy of clustering.

Here, this embodiment is applicable to the sequencing result related to the sample obtained by the HiCEP. In the case of the data related to the HiCEP, the fragment lengths are set to the same length by the adaptor sequences. This allows obtaining a good clustering result, and this embodiment especially provides the effects.

It should be noted that the present invention is not completely limited to the embodiments described above, and modifications of components can be made at the stage of carrying out the invention without departing from the spirit thereof. Further, various inventions can be made by properly combining the components disclosed in the embodiments described above. For example, when the problems described in the section of PROBLEMS TO BE SOLVED BY THE INVENTION can be solved and the advantages of the invention can be obtained even if some of all the components shown in the embodiments are eliminated, a configuration in which those components are eliminated can also be extracted as an invention. Moreover, components in different embodiments may be suitably combined together.

## Claims

1. A method for determining whether a difference between a first reference sequence and a plurality of first comparative sequences sharing homology with the first reference sequence is caused by a mutation of the first comparative sequence or caused by a read error in sequencing, the method comprising:
generating respective second comparative sequences by substituting a sequence having a predetermined base number or more of consecutive same bases for a sequence having the predetermined base number of the consecutive same bases in the respective first comparative sequences;
generating a second reference sequence by substituting a sequence having the predetermined base number or more of consecutive same bases for a sequence having the predetermined base number of the consecutive same bases in the first reference sequence;
calculating a plurality of edit distances after shortening as edit distances of the respective second comparative sequences with respect to the second reference sequence;
calculating an average value after shortening as an average value of the plurality of edit distances after shortening; and
determining whether the difference is caused by the mutation or caused by the read error based on the average value after shortening.

2. The method according to claim 1, further comprising:
calculating a plurality of edit distances before shortening as edit distances of the respective first comparative sequences with respect to the first reference sequence; and
calculating an average value before shortening as an average value of the plurality of edit distances before shortening, wherein
the determining includes determining whether the difference is caused by the mutation or caused by the read error based on a relationship between the average value before shortening and the average value after shortening.

3. The method according to claim 2, wherein
the determining includes determining that the difference is caused by the mutation when the average value after shortening is within a predetermined average value range or when the average value after shortening is larger than the average value range and the average value before shortening is within the average value range.

4. The method according to claim 2, wherein
the determining includes determining that the difference is caused by the mutation when both a first condition and a second condition are satisfied,
the first condition is that the average value after shortening is within a predetermined average value range or that the average value after shortening is larger than the average value range and the average value before shortening is within the average value range, and
the second condition is that a proportion of a number of the second comparative sequences having a difference between the second reference sequence and the second comparative sequence with respect to a number of all the first comparative sequences is within a predetermined sequence number range.

5. The method according to any one of claims 1 to 4, wherein
the predetermined base number is 2.

6. The method according to claim 3 or 4, wherein
the average value range is a range including 0.5.

7. The method according to claim 4, wherein
the sequence number range is a range including 50%.

8. The method according to any one of claims 1 to 4, wherein
the first reference sequence is a consensus sequence obtained based on an identity of the first comparative sequence.

9. The method according to any one of claims 1 to 4, for further causing the computer to execute
performing clustering of the plurality of first comparative sequences corresponding to the difference when the difference is determined to be caused by the mutation.

10. The method according to any one of claims 2 to 4, wherein
the first comparative sequence is a base sequence sandwiched between two adaptor sequences to be used in HiCEP,
the calculating the edit distances after shortening is to calculate a plurality of edit distances after shortening as edit distances of the respective second comparative sequences with respect to the second reference sequence, regarding a predetermined number of bases using an end of the adaptor as a reference, and
the calculating the edit distances before shortening is to calculate a plurality of edit distances before shortening as edit distances of the respective first comparative sequences with respect to the first reference sequence corresponding to the base sequence after the calculation of the edit distance after shortening.

11. A method for determining whether a difference between a first reference sequence and a first comparative sequence sharing homology with the first reference sequence is caused by a mutation of the first comparative sequence or caused by a read error in sequencing, the method comprising:
generating a second comparative sequence by substituting a sequence having a predetermined base number or more of consecutive same bases for a sequence having the predetermined base number of the consecutive same bases in the first comparative sequence;
generating a second reference sequence by substituting a sequence having the predetermined base number or more of consecutive same bases for a sequence having the predetermined base number of the consecutive same bases in the first reference sequence;
calculating an edit distance of the second comparative sequence with respect to the second reference sequence; and
determining whether the difference is caused by the mutation or caused by the read error based on the edit distance.
